# EUROPEAN PATENT SPECIFICATION

(11) **EP 3 102 266 B1**
(45) Date of publication and mention of the grant of the patent: **30.10.2019**
(21) Application number: 14819045.7
(22) Date of filing: 25.11.2014
(51) Int. Cl.: A61M 15/00, A24F 47/00

(54) **PULMONARY DELIVERY DEVICES**
VORRICHTUNGEN ZUR PULMONALEN VERABREICHUNG
DISPOSITIFS D'ADMINISTRATION PULMONAIRE

(30) Priority: 26.11.2013 GB 201320834; 04.03.2014 GB 201403827; 04.03.2014 GB 201403828; 02.04.2014 GB 201405979
(43) Date of publication of application: 14.12.2016
(73) Proprietor: Twenty Sixteen (2016) Pharma Limited, Liverpool, Merseyside L3 5TF (GB)
(72) Inventor: PANDYA, Anant, Croydon CR0 5DJ (GB); GALLAGHER, George, Caerwys CH7 5PY (GB)
(74) Representative: Lees, Kate Jane
(86) International application number: PCT/GB2014/000484
(87) International publication number: WO 2015/079198

(56) References cited:
- WO-A1-2013/152873
- DE-A1- 19 600 409
- US-A- 4 253 453

## Description

This invention relates to pulmonary delivery devices, and in particular, but without limitation, to pulmonary delivery devices suitable for delivering active molecules and/or medicaments to users. Where the word medicament is used herein, it should be construed to include any active molecule, including a medicament, drug, flavouring, foodstuff etc.

Pulmonary delivery devices have widespread uses in modern medicine as they enable drugs and medicaments to be delivered directly to the user's lungs. Moreover, as medicaments delivered to the lungs enter the bloodstream directly, rather than via the body's metabolism, as is the case with injected, oral or pessary delivery systems, the benefits of the medicament, especially in pain relief or drug-weaning applications, are felt by the user almost immediately. Another major benefit of pulmonary delivery devices is their ability to deliver drugs without the use of needles. In addition the pulmonary delivery device bypasses the liver i.e. first pass metabolism thus enabling lower dose and faster action.

Existing pulmonary delivery systems take various forms, including inhalator sprays and vapour delivery systems whereby the medicament is admixed to an inhalable vapour (often a water-containing vapour). This invention relates to the latter type of pulmonary delivery devices. WO2013/152873 describes an aerosol generating device comprising two reservoirs for generating an aerosol.

A vapour inhaler-type pulmonary delivery system comprises a carrier liquid, often water or a water-glycol mixture (the glycol serving to stabilise the water droplets when in the vapour form) to which is admixed a desired medicament. The carrier liquid can be vaporised in various ways, such as by spraying it through a nozzle, but in many cases, it is simply heated to form a vapour comprising the carrier liquid and the desired medicament. The resultant vapour is then inhaled by a user to deliver the medicament.

There are many problems associated with existing vapour-phase pulmonary delivery devices, chief amongst which is controlling the amount of medicament delivered in each dose. Errors in dosing can arise through intrinsic factors, such as the composition, stability and longevity of the carrier liquid-medicament mixture/solution, as well as extrinsic factors, such as the inhalation capacity and frequency of the user. As such, it is virtually impossible, with known vapour-phase pulmonary delivery devices, to accurately control the dose delivered in each use, but also to regulate and/or control the frequency of use by a user. The lack of precise dose control has led to questions surrounding the efficacy and/or safety of existing pulmonary delivery systems and this invention aims to provide a solution to one or more of the above problems. Additionally or alternatively, the invention aims to provide an alternative vapour-phase pulmonary delivery device.

An aspect of the invention provides a pulmonary delivery apparatus comprising: a first vaporiser adapted to vaporise a quantity of a first liquid, and being characterised by: a second vaporiser adapted to vaporise a quantity of a second liquid, the second liquid being a different liquid to the first liquid; and an outlet via which, in use, a user can inhale a mixture of the first and second vapours.

By providing two vaporisers, rather than just one, as is the case in existing vapour-phase pulmonary delivery devices, it is possible to control the composition of the mixture by controlling the quantity of vapour released from each of the vaporisers. Suitably, the delivery apparatus comprises a controller adapted to control, in use, the composition of the first and second vapours in the mixture, that is to say, by controlling the relative amounts of the first and second vapours in the mixture, or the ratio between the two. The controller could be adapted to switch one or the other of the vaporisers on or off, thus providing the option of delivering one or the other of the liquids in vapour form.

In an example, the first liquid comprises a carrier liquid (i.e. a liquid capable of forming a stable vapour), which may be an inert (non-medicated carrier liquid), such as water, or a water-glycol mixture. The second liquid, in an example can comprise the desired medicament. Thus, if the device is configured to deliver only the first liquid in vapour form to the user, the user receives a zero dose of the medicament. On the other hand, if the ratio of the first and second liquids is adjusted to a non-unity value, an amount of the second liquid can be added to the mixture inhaled by the user, thus delivering a dose of the medicament.

In another embodiment, the first and second liquids comprise different medicaments, and so the device can be adapted to vary the relative concentrations of the two, which may be particularly useful where, say, a course of drugs includes an initial dose of a first medicament, which transitions to a dose of a second medicament over a period of time.

Suitably, by enabling the device to control the ratio of one liquid to the other in the mixture, it is possible to vary the composition for the mixture in infinitesimal increments from 100% of the first liquid to 100% of the second liquid. Of course, if more than two vaporisers are provided in the device, then the ratios could be adjusted accordingly.

The vaporiser suitably comprises an electric heater, for example a battery-powered resistive heating wire or coil. The current delivered to the resistive heating wire or coil can be used to control the temperature of the wire or coil, and thus regulate and/or control the heating and vaporisation of the liquids. In example, the heater comprises a hydrophilic or super-hydrophilic foil, which coats with a film of the liquid to be vaporised. A current can be passed through the coil to heat it, thereby vaporising the liquid.

In an example, the heaters of the two vaporisers could be of different types, for example, one coil heater and one foil heater. This advantageously provides more precise control of the liquid being evaporated by the foil heater, say, and less controlled, or uncontrolled, evaporation of the liquid being vaporised by the coil heater.

A feedback circuit is suitably provided to thermostatically regulate the temperature, or temperature profile of the heater. For example, a circuit may be provided to monitor the resistance of the wire or coil (the resistance being dependent on the wire or coil's temperature) and to adjust the current in the wire or coil such that the resistance, and hence the temperature, is controlled.

An example provides a vaporiser suitable for use in a pulmonary delivery device, comprising an electric heater adapted to vaporise a quantity of vaporisable liquid in contact therewith, the vaporiser further comprising a circuit configured to apply a time-dependent heating and/or cooling profile by temporally controlling an electric current in the heater in response to a measured temperature thereof.

Such a configuration, that is to say, a time-dependent heating and/or cooling profile, suitably controls the vaporisation of the liquid or liquids more precisely and reproducibly, and/or improves the longevity of the heater, which is suitably a heating wire, foil or coil.

Other heating devices could equally be used, such as thermionic emitters, Peltier devices, infrared emitters and so forth, and the invention is not restricted to resistive heater wires, foils or coils.

Additionally, or alternatively, the liquids could be vaporised by atomising or forcing a liquid through an aperture to form a stable vapour or mist. In an example, one of the vaporisers comprises a thermal (heat-based) vaporiser adapted to evaporate the respective liquid, whereas another of the vaporisers comprises an atomiser adapted to form a mist of the respective liquid by forcing it through a nozzle or aperture.

Suitably, the device comprises a separate vaporiser associated with each of the liquids, or a single vaporiser adapted to vaporise more than one of the liquids simultaneously.

An aspect of the invention comprises a pulmonary delivery apparatus comprising: a first vaporiser adapted to vaporise a quantity of a first liquid, and being characterised by: a second vaporiser adapted to vaporise a quantity of a second liquid, the second liquid being a different liquid to the first liquid; and an outlet via which, in use, a user can inhale a mixture of the first and second vapours.

The controller for controlling the ratio of the first and second liquids in the mixture is suitably programmable to change the ratio on a dose-by-dose basis. This may be accomplished using a microprocessor. In an example, the controller comprises a computer, such as a System On Chip (SOC) device, which executes an application that controls the ratio. The computer is suitably adapted to interface with an external programming device. Suitably, the external programming device comprises a computer, which communicates with the device via a physical comms port (e.g. a USB port) or via a virtual comms port (e.g. a Wi-Fi, Bluetooth^{RTM} or WAN port).

In certain examples, the device can be preconfigured with a number of user-selectable programs. For example, the device may comprise a selector switch enabling the user to select between, say: 1) 100% of the first liquid and 0% of the second liquid; 2) 0% of the first liquid and 100% of the second liquid; and 3) a selected ratio of the two liquids, for example a 50%50% mix, a 90%-10% mix, and so on.

In another example, the first vaporiser is always on, whereas the operation of the second vaporiser is user-initiated, for example, using a push button on the device.

Suitably, an interface is provided that enables a user, physician or health professional to configure the device to operate in various modes. The interface suitably comprises an application executed in an external computer, which is adapted to communicate with the device and thus to control and/or program it.

For example, a nicotine weaning device, the pulmonary delivery system resembles a cigarette, a pipe or a cigar. In such a situation, the first liquid can comprise an inert mixture of water and glycol and the second liquid can contain a mixture of a carrier liquid (again possibly a water-glycol mixture) and the desired medicament, in this case, liquid nicotine. The device can thus be programmed to deliver a certain dose of medicament (nicotine) in each "puff" of the device, or over a given period, such as a day.

In the former case, the ratio of the first and second liquids is configured such that the inert liquid is always dispensed, whereas the dose of the second liquid is controlled to deliver only the pre-determined dose in each "puff". Thus, no matter how long, or how hard, the user draws on the device, only the predetermined amount of medicament (nicotine in this example) is delivered each time. This configuration usefully addresses the extrinsic dosage variations that arise from variations in users' physiology.

In the latter case, the dose can be configured to deliver a dose over a period of time, such as a day. In this case, the ratio of the first and second liquids can be dynamically configured such that for a given number of "puffs" per unit time, the user will be administered the predetermined dose.

In an example, the timing of the "dose" can be configured. In one example, the device is configured to permit unlimited use of a first one of the liquids, but a metered and/or dosed consumption of the second liquid. The metering and/or dosing is suitably time-dependent, for example, allowing dosing two, three (or any desired number of) times per day, at specific times. The dose can also be configured to vary through the course of a day, for example, with higher doses in the morning and/or evening, say.

However, the device is suitably configured to monitor the number of puffs on the device, and to adjust the ratio of the first and second liquids to accord. Thus, the dose-per-puff of the medicament can be increased if the user is under-using the device, or reduced if the user is overusing the device, thus ensuring that the predetermined dose is not exceeded in the time interval. Such a configuration can usefully be used to ensure compliance with a prescribed dosage regimen, thereby negating the effects of poor user discipline. If the user under-uses the device, then the dose-per-puff is increased to compensate. Conversely, if the user over-uses the device, the dose-per-puff can be reduced to compensate. If the user over-uses the device excessively, and reaches the maximum dose level, the medicated liquid can be switched off, thereby allowing the user to continue to use the device, albeit in an inert mode, with no further delivery of medicament.

The device suitably logs and/or monitors the user's habits, and uses this information to re-configure the dosage in future time intervals. As such, an estimate of the number of puffs per day, say, can be adjusted up or down automatically, based on historic usage data. This usage data can also be fed back to health professionals, if desired, via the computer interface for reporting and/or monitoring purposes.

The or each vaporiser suitably comprises a reservoir for retaining, in use, a quantity of the respective liquid and a conveyor adapted to convey, in use, the liquid from the reservoir to a heater.

In an example, the reservoir comprises a vial and the conveyor comprises a wick extending between the interior of the vial and the heater. Suitably, a resistive heating wire, such as that described herein, can be wrapped or coiled around the wick to vaporise the liquid.

The conveyor may comprise a capillary tube extending between the vial and the heater. In other examples, the conveyor comprises the piston of a syringe and the reservoir comprises the cylinder of a syringe. If the syringe comprises a mechanically-actuated syringe, such as a motorised syringe, the volume of liquid dispensed can be controlled by controlling the movement of the piston within the cylinder.

In another example, the vaporiser comprises a reservoir containing a liquid under pressure that can be forced through an aperture to form a stable vapour. In an embodiment, this could comprise a pressure vessel containing the liquid and a propellant, and the conveyor comprises a flow control valve associated with the outlet of the pressure vessel. By opening the flow control vale for a predetermined interval, a predetermined quantity of the liquid can be dispensed.

The reservoir and/or conveyor is suitably configured such that the size of the reservoir, the pressure within the reservoir and the size of the reservoir outlet at its narrowest point are arranged such that the peak volumetric flow rate of the liquid for each dose with the valve fully open has a variation of less than 10%.

The first and second liquids suitably comprise a solvent and a stabiliser. The stabiliser is suitably adapted to stabilise droplets of the solvent in air. The carrier liquid can comprise any one of more of the group comprising:
solubilizers, solvents and mixtures thereof such as water, alcohols such as glycerol, propylene glycol, polyethylene glycol, vegetable oils, mineral oils, lipids, cyclodextrins etc. Surface active agents such as anionic agents with carboxylate ions, sulphate groups and sulphonate groups; cationic surfactants, nonionic surfactants such as polyol ethers, polyoxyethylene esters and ethers, poloxamers; amphoteric surfactants, natural emulsifiers, sucrose esters and alkylpolyglucosides;
antioxidants such as ascorbic acid and its salts and derivatives tocopherols (vitamin E), thiol derivatives such as cysteine and acetyl cysteine, butylated hydroxyanisole (BHA), butylated hydroxytoluene (BHT), sodium hydrogen sulfite, sodium metabisulfite, sodium thiosulfate;
absorption enhancers such as alcohols, azone;
chelating agents, such as EDTA and galates;
minerals, such as fluorides;
propellants, such hydrofluroalkanes (HFA) Chlorofluorocarbons (CFCs), carbon dioxide etc.; sweeteners such as artificial sweeteners e g saccharin and its sodium and calcium salts, aspartame, acesulfame and its potassium salt, thaumatin and glycyrrhizin, polyhydric alcohols such as sorbitol, xylitol, mannitol and glycerol, glucose, fructose, galactose, sucrose, lactose, maltose and mixtures thereof;
flavourings and aroma such as essential oils of dried flowers, buds, leaves, stems, fruit, seeds, peel, bark, or root e g oil of peppermint, spearmint, eucalyptus, wintergreen, clove, cardamom, cinnamon, bitter almond, coriander, caraway, ginger, juniper, orange, bitter orange, lemon, grapefruit, bergamot, thyme, fennel rosemary etc., natural flavors and aroma agents of essential oils or concentrates of flavor components with natural origin from e g fruits, berries, nuts, spices, mints, tobacco, cocoa, coffee, tea, vanilla, liquorice, caramel, toffee, honey, wine, liquors and brews, synthetic flavors and aroma agents consisting of mixtures of chemicals comprising hydrocarbons, alcohols, aldehydes, esters, ketones, ethers and oxides blended to match the natural flavor of e g fruits, berries, nuts, spices, mints, tobacco, cocoa, coffee, tea, vanilla, liquorice, caramel, toffee, honey, wine, liquors or brews and mixtures thereof; and
pH regulators and buffering agents such as sodium, potassium or calcium hydroxide; bicarbonates, citrates and phosphates, etc.

One or both of the first or second liquids suitably comprises a active molecule or medicament. The active molecule, excipient or medicament may comprise any one or more of the pharmacologically active compounds from the group comprising:
H2-receptor antagonists such as cimetidine; and ranitidine;
Prostaglandin analogues such as misoprosol;
Proton pump inhibitors such as lansoprazole; omeprazole; and pantaprazole;
Agents to treat food allergies such as sodium cromoglicate;
Cardiac glycosides such as digoxin;
Diuretics such as amiloride; bendroflumethizide; indapamide; furosemide; hydrochlorothiazide; and xipamide;
Drugs for arrythmias such as procainamide; lidocaine; propranolol; atenolol; bisoprolol; carvedilol; pindolol; and nebivolol;
Antihypertensives and agents for treatment of angina such as clizapril; lisinopril; ramipril; trandolapril; amlodepine losartan; glyceryl trinitrate, isosorbide mononitrate; amlodipine; diltiazem; felodipine; isradipine; lacidipine etc.;
Lipid regulating drugs such as statins;
Drugs acting on the respiratory system such as salbutamol; terbutaline; bambuterol;
Antihistamines such as cinnarazine; promethazine; perphenazine and prochlorprazine;
Hypnotics such as zolpidem; zopiclone; clomethiazole;
Anxiolytics such as benzodizapines; buspirone;
Antipsychotic agents such as benperidol;fluphenazine; pimozide and amisulpride;
Antidepressant drugs such as tricyclics; mianserin; MAOIs; SRIs; reboxetine etc.;
Central nervous system (CNS) stimulants such as methylphenidate;
Drugs used in the treatment of nausea such as antihistamines; domperidone; metoclopramide; 5HT3 antagonists; hyoscine, and betahistine;
Opiod analgesics such as morphine; buprenorphine and fentanyl;
Anti-migraine drugs such as 5HT1 agonist and ergot alkaloids;
Drugs used in treatment of Parkinsonism such as apomorphine; bromocriptine; lisuride; haloperidol and ergot alkaloids;
Drugs used in substance dependence such as nicotine and buprenorphine;
Drugs used in dementia such as rivastigmine; dihydroergotamine; dihydroergocristine and dihydroergocryptine;
Antibiotics; antifungals; antivirals and antimalarials;
Drugs used in treatment of diabetes;
Glucocorticoid therapy using steroids such as betamathasone and dexamethasone;
Male and/or female sex hormones such as estradiol; norethisterone; progesterone; testosterone and esters;
Pituitary hormones such as vasopressin and desmopresin;
Drugs affecting bone metabolism such as calcitonin and bisphosphonates;
Endocrine drugs such as bromocriptine and cabergoline;
Contraceptives such as oetrogens; progestrogens and combinations thereof;
Drugs used in urinary frequency and enuresis such as oxybutinin and desmopressin;
Drugs used in erectile dysfunction such as apomorphine and sildenafil;
Drugs used in malignant disease and immunosuppresion such as buslfan; antimetabolites; alkaloids; corticosteriods; hormones and interferons;
Non-steroidal anti-inflammatory drugs such as diclofenac; piroxicam and refoxicab;
Drugs used in treatment of gout such as colchicines;
Drugs used in neuromuscular disorders such as neostigmine and pyridostigmine;
Muscle relaxants such as diazepam; tizanidine;
Vaccines delivered by subcutaneous route; and
Agents for the treatment of nicotine withdrawal symptoms such as nicotine.

The or at least one active compound may be a nutraceutically active compound. A "nutraceutically active compound" is a compound, derived from a natural origin (animal or vegetable) that has a beneficial and/or therapeutic effect on the human or animal body in the treatment of a condition. Such compounds may be regarded as nutrients.

Suitable nutraceutically active compounds may be natural products extracted from animals or vegetables. Examples of suitable nutraceutically active compounds include:
Carotenoids such as lycopene, lutein, astaxanthin and β-carotene; glucosamine or Nacylglucosamine; ubiquinone;
Vitamins such as vitamins A, C, D and E; Rosmarinic acid; Honokiol; Magnolol; Chlorogenic acid; Oleuropein; Methylsulphonylmethane ("MSM"); Collagen and Chondroitin; Boswellin and boswellic acid; Escin and esculin; Tumeric extracts such as curcuminoids and etrahydrocurcuminoids; Gingerol and gingerone; Triterpenes such as ursolic acid and oleanolic acid; Diterpenes such as asiaticoside, sericoside and ruscogenins; Hydroxycitric acid ("HCA") and niacinamide hydroxycitrate;Trigonellin; and Corosolic acid; Saw palmetto; and St John's Wort.

The device suitably comprises a battery, such as a rechargeable battery, for powering the heater and or control circuitry.

Where the device comprises more than one heater, each heater may be controlled independently to control the vaporisation rate of the respective liquids, thus controlling the relative proportions of each in the mixture. A first one of the vaporisers may be uncontrolled, whereas the quantity of the second liquid can be dosed.

The heater is suitably switched on or off using a switch. The switch is suitably an automatic switch that is triggered by the user inhaling on the device. The switch may therefore comprise a pressure-activated switch associated with the outlet of the device, whereby when a user draws on the device, the switch is turned on thereby automatically switching on the heater, and whereby the heater is switched off again when the user ceases drawing on the device. Suitably, the device additionally comprises a second pressure-sensitive switch for monitoring the pressure of the ambient air.

Another aspect provides a pulmonary delivery device comprising a vaporiser and an outlet aperture through which, in use, vapour can be inhaled by a user, a first pressure-sensitive switch communicating with the outlet aperture and being adapted to detect a drop in pressure corresponding to the user inhaling on the device, a controller adapted to turn on the vaporiser in response to the triggering of the first pressure-sensitive switch, characterised by a second pressure-sensitive switch adapted to sense the ambient pressure surrounding the device, wherein the controller is configured to turn on the vaporiser when only the first pressure-sensitive switch is actuated.

This configuration is useful, from a practical point of view, as it can be used to prevent inadvertent triggering of the heater. For example, when the user intends to trigger a dose, he or she can suck on the inlet, thereby triggering the first switch, but not the second. However, when, say, there is a sudden change in ambient air pressure (for example, when entering a train tunnel), both pressure switches will detect this change, indicating that a dose is not needed. Thus, the device can be configured to operate when only the pressure-switch in communication with the device's outlet is triggered.

Another aspect provides a pulmonary delivery apparatus comprising: a first reservoir for retaining, in use, a quantity of a first liquid; a first conveyor adapted to convey, in use, the first liquid from the first reservoir to a heater adapted, in use, to heat a quantity of the first liquid to form a first vapour; characterised by: a second reservoir for retaining, in use, a quantity of a second liquid different from the first liquid; a second conveyor adapted to convey, in use, the second liquid from the first reservoir to a heater adapted, in use, to heat a quantity of the second liquid to form a second vapour; and an outlet through which, in use, a user can extract, by inhalation, a mixture of the first and second vapours from the apparatus.

Embodiments of the invention shall now be described, by way of example only, with reference to the accompanying drawings in which:
Figure 1 is a perspective view of a pulmonary delivery device in accordance with the invention;
Figure 2 is an exploded view of the pulmonary delivery device of Figure 1;
Figure 3 is a close up view of part of Figure 2;
Figure 4 is a schematic view of another pulmonary delivery device in accordance with the invention;
Figure 5 is a schematic system diagram showing an interface for the pulmonary delivery device of the invention; and
Figure 6 is a perspective view of an embodiment of a simplified pulmonary delivery device in accordance with the invention.

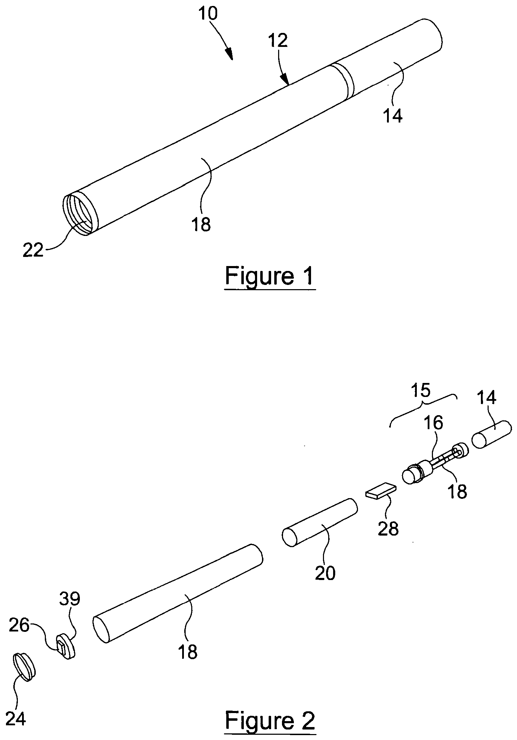

In Figures 1, 2 and 3, a pulmonary delivery device 10 comprises a generally cylindrical main body portion 12 adapted to resemble a cigarette. The main body portion 12 comprises a tubular filter chamber 14 encasing a vaporiser 15, and a tubular battery chamber 18 encasing a rechargeable battery 20. The tip 22 of the main body 12 is closed off by a translucent end cap 24, behind which sits an LED indicator light 26 that illuminates when the device 10 is in use. A control circuit 28 is contained within the body 12, which comprises a programmable circuit for controlling the operation of the device 10, in use.

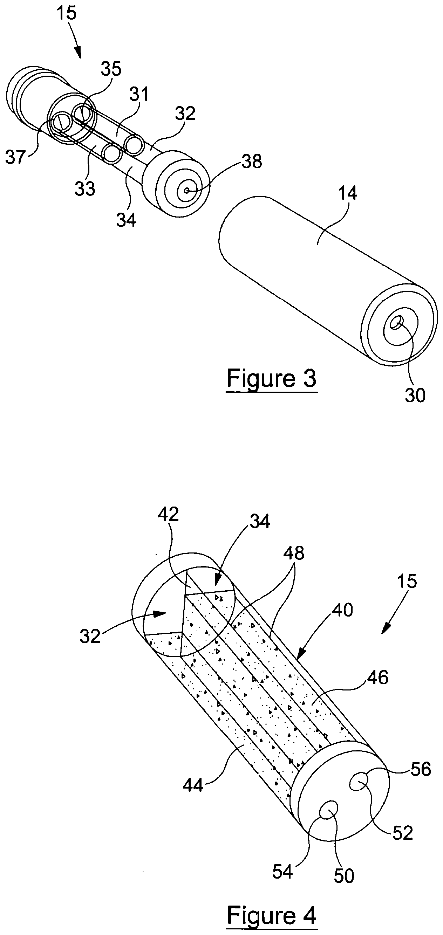

Turning to Figures 2 and 3, the device 10 comprises a first pressure sensor (not visible) located within the filter chamber 14, which has an outlet aperture 30 therein through which, in use, vapour generated by the device 10 can be inhaled by a user. When the user draws on the filter chamber 14, the pressure sensor (not visible) turns on the heater within the vaporiser 15 to form a vapour comprising the first and/or second liquids, to be inhaled.

The vaporiser 15 comprises a pair of reservoirs 32, 34 which contain the separate first and second liquids. Each reservoir 32, 34 comprises a capillary wick 31, 33, which absorbs the liquid, and whose ends touch a separate heater element in the form pyramid-shaped, super-hydrophilic foils 35, 37, which are wetted by the respective liquids, in use.. The heater elements 35, 37 could alternatively comprise a restive heating coil, which is wrapped around the wicks 31, 33. In any event, the heater elements 35, 37 are connected to the battery 20 under the control of the control circuit 28.

When the heaters 35, 37 are switched on, the liquid thereon is evaporated, and forms a vapour within the interior of the vaporiser 15. The vaporiser 15 has an outlet aperture 38, which communicates with the interior of the filter tube 14 thus allowing the vapours to mix in the hollow space therein, before being inhaled by the user, via the outlet aperture 30 of the device.

The control circuit 28 comprises a resistance sensor operatively connected to each heater element 35, 37, which measures the heater's resistance, and infers from that, the heater temperature. The control circuit 28 additionally comprises a current limiting circuit for limiting the current to the heaters 35, 37 and is programmed to heat them according to a predetermined, time-dependent heating/cooling profile.

When a user draws on the filter chamber 14, the pressure switch (not visible) triggers the control circuit 28 to heat the heaters 35, 37. The control circuit 28 thereby connects the battery 20 to the heaters 37, 37 in a controlled and reproducible manner. As such, the time, and time-at-temperature of the heaters 35, 37 is thus controlled, thereby regulating the vaporisation of the liquids from their respective wicks 38, 40.

The control circuit 28 is also operatively connected to a second pressure sensor 39 (Figure 2), which measures the ambient air pressure. The control circuit 28 is configured to switch on the heaters 35, 37 only when the first pressure switch is triggered, as described hereinabove.

A vapour is thereby formed adjacent the heater within a hollow interior space (a mixing chamber) located towards the tip of the filter chamber 14, i.e. the space between the vaporiser chamber 36 and the outlet aperture 30, when the device 10 is assembled.

The control circuit 28 is adapted to vary the current, and hence the temperature and time-at-temperature of each of the heaters 35, 37. Thus, provided the liquids vaporise according to a predetermined model, which is a fair assumption given sufficient experimentation, the control circuit 28 is able to control the composition of the mixture by adjusting the amount of each liquid vaporised. The ratio of the liquids in the resultant mixture can be adjusted by the control circuit 28, in accordance with a pre-programmed dosing regimen.

Figure 4 shows an alternative vaporiser 15 in accordance with the invention. In Figure 4, the vaporiser 15 comprises a hollow cylindrical pressure vessel 40 comprising a central divider 42 thus dividing the interior thereof into two separate reservoirs 32, 34 for first 44 and second 46 liquids. A pressurised propellant gas 48 occupies the remaining space of each reservoir 32, 34 and an outlet aperture 50, 52 is provided to enable the liquids 44, 46 to escape from their respective reservoirs under the action of the pressurised propellant 48. Each outlet aperture 50, 52 is sufficiently small as to control (mass-limit) the amount of liquid 44, 46 that can escape in each dispensation, and is selectively closed and/or opened by a respective control valve 54, 56.

The control valves 54, 56 are each independently connected to the control circuit 28 enabling them to be controlled independently. The control circuit 28 can thus be configured to open each valve 54, 56 a given number of times, per actuation, thereby incrementally controlling the dose of each liquid dispensed (the dose per actuation being constant due to the size of the outlet apertures 50, 52). Thus, the device 10 is able to accurately control the ratio of the first 44 and second 46 liquids dispensed in each actuation, and hence the dose of a particular medicament of mixture of medicaments in the first 44 and second 46 liquids.

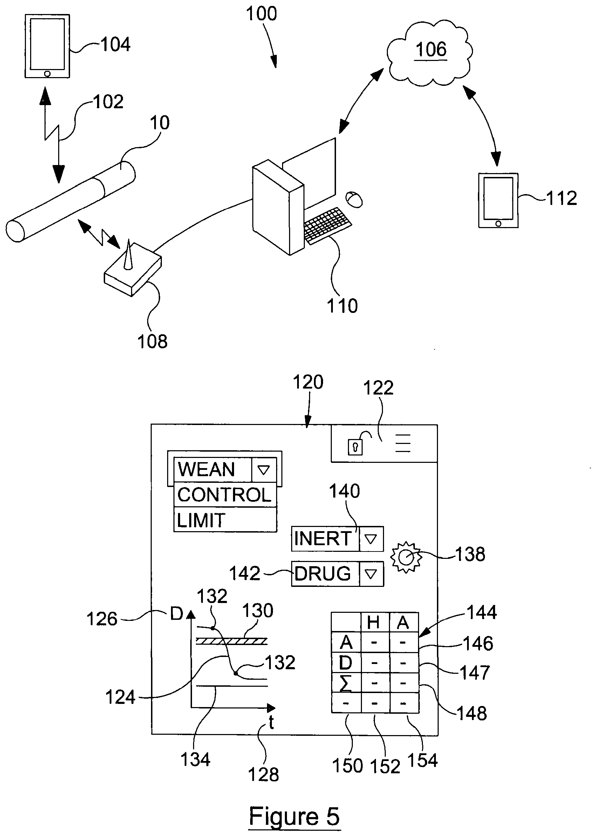

A dose control system 100 is shown in Figure 5 of the drawings. In Figure 5, a pulmonary delivery device 10 according to the invention is wirelessly connected 102 to a user's smartphone, tablet computer or PC 104 and to the internet 106, via a Wi-Fi access point 108, such as a broadband router. Internet connected computers 110, 112 (local or remote) can thus connect to the device 10 wirelessly, as can the user him or herself. The wireless connection is provided via a Wi-Fi and/or Bluetooth^{RTM} interface of the control circuit 28, thereby providing a graphical user interface (GUI) 120 on any of the devices 104, 110, 112 for interacting with the device 10.

The GUI 120 has a secure login-in system 122 to prevent unauthorised re-configuration of the device 10 and allows a user to select between three main modes of operation, namely a "wean" mode 124 whereby the dose 126 of a given medicament can be reduced over time 128, as shown on a dose-time graph 130 of the GUI. The graph has dragable handles 132 that enable the shape of the curve to be adjusted to change the weaning profile 124, i.e. the severity, duration, delay etc. of the weaning process.

Another option from the drop-down menu is to is to select a control program 134, which ensures that a desired quantity of medicament is administered over a period of time. The dose-per-puff is thus controlled to ensure, on average, a relatively even administration of the medicament over the time period.

A third option is to set an upper limit 136, which may be useful in analgesic applications. This program prevents a maximum dose per unit time from being delivered, but allows for under-administration.

The GUI 120 comprises a configuration settings menu 138, which enables a user to configure the GUI in accordance with the liquids 140, 142 in the device. A history table 144 is also provided, which provides a summary of the number of administrations 146, the amount of medicament delivered 147 and a running total 148. These data are shown on a historic 150, actual 152 and a target 154 basis to facilitate monitoring of the drug delivery to the user.

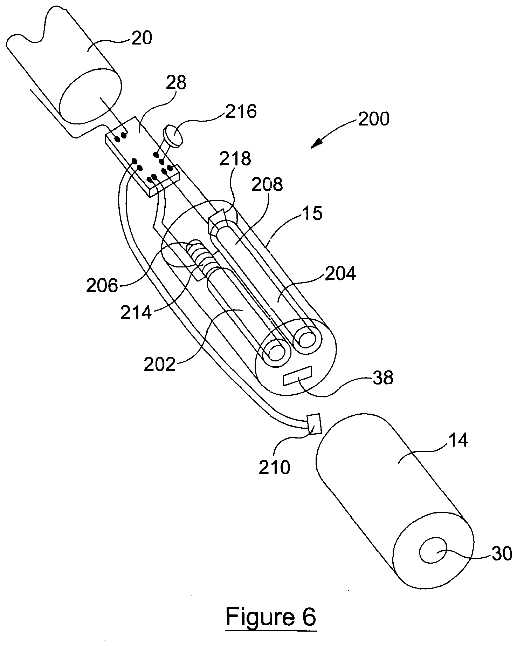

A simplified version of the invention is shown, schematically, in Figure 6 of the drawings, in which a pulmonary delivery device 200 comprises a battery 20, vaporiser 15 and a filter tube 14, as previously described. The vaporiser 15 is largely similar to that shown in Figures 1 to 3 of the drawings, but with the following differences:

The vaporiser 15 comprises two reservoirs 202, 204 for containing respective quantities of first and second liquids (not shown). Each reservoir 202, 204 has a respective wick 206, 208, which conveys the liquids to the heaters of the vaporiser 15.

The first reservoir 202 contains an inert liquid, such as a water-glycol mixture, which forms an inhalable vapour that can be consumed without restriction by a user. The device 200 has a pressure switch 210, which detects when a user inhales on the device 200. The pressure switch 210 is connected to the control circuit 28, via wires 212, and the control circuit is adapted to switch on a current, from the battery 20, to a resistive heating coil 214 wrapped around an end of the wick 206, which evaporated the first liquid to form a vapour that can be drawn from the device 200, via the vaporiser outlet 38 and the device's main outlet aperture 30.

The device 200 additionally comprises a push switch 216 that is accessible from the exterior of the device 200, which a user can depress, in use, to turn on the second heater 218, which, in the illustrated example, is a super-hydrophilic foil heater in contact with the second wick 208. Thus, a user can use the device 200 at will, and can choose when to administer a dose of a medicament, which is contained in the second reservoir 204, by pressing on the button 216 during inhalation. The advantage of the configuration shown is that the use of a super-hydrophilic foil heater 218 enables the evaporation of the second liquid (containing the drug) to be carefully, and reproducibly controlled. However, the complexity and cost of the foil heater is not duplicated for the second, inert, liquid that does not require such a high level of control: thus a simpler and cheaper (in this case, a resistive wire coil) can be used for the uncontrolled, or un-limited liquid. This reduces the cost and complexity of the device 200, overall.

The invention is not restricted to the details of the foregoing embodiments, which are merely exemplary of the invention. For example, the shape and configuration of the device can be changed, the materials of manufacture, the combinations of vaporiser technology used, the combinations of heaters used, the additional features, such as the on/off switch, etc. can be varied without departing from the invention, as long as they fall within the scope of the claims.

## Claims

1. A pulmonary delivery apparatus comprising: a first vaporiser adapted to vaporise a quantity of a first liquid, and a second vaporiser adapted to vaporise a quantity of a second liquid, the second liquid being a different liquid to the first liquid and wherein one or both the first or second liquids comprises an active molecule, excipient or medicament; and an outlet via which, in use, a user can inhale a mixture of the first and second vapours and being **characterised by**: a programmable controller adapted to automatically control, in use, the ratio of the first and second liquids in the mixture to change the ratio of the liquids on a dose dependent basis.

2. The pulmonary delivery device of claim 1, wherein the controller is preconfigured with a number of user-selectable modes of operation, and wherein the device comprises a selector switch adapted to cycle the controller between the different modes of operation, preferably wherein user-selectable modes of operation comprise a first mode in which the mixture comprises 100% of the first liquid and 0% of the second liquid; and a second mode in which a selected ratio of the two liquids is present in the mixture.

3. The pulmonary delivery device of claim 1 or claim 2, wherein the first vaporiser is always activated and wherein the second vaporiser is selectively activated by a button of the device.

4. The pulmonary delivery device of claim 1, 2 or 3, wherein the controller is adapted to dispense a particular dose of one of the liquids per inhalation; and/or wherein the controller is adapted to dispense a particular cumulative dose of one of the liquids per unit time; and/or wherein the controller is adapted to dispense a particular dose of one of the liquids only at predetermined times of day, and to inhibit or prevent dispensing the said liquid at other times of day; and/or wherein the controller is adapted to permit unlimited consumption of a first one of the liquids, but a metered and/or dosed consumption of a second one of the liquids.

5. The pulmonary delivery device of claim 1, wherein the controller is adapted to adjust the ratio of the first and second liquids in the mixture to compensate for over-or under-use of the device.

6. The pulmonary delivery device of any preceding claim, wherein one or more of the vaporisers comprises an atomiser, preferably wherein the atomiser comprises a reservoir containing a liquid under pressure that can be forced through an outlet aperture to form a stable vapour and a flow control valve for selectively opening and closing the outlet aperture.

7. The pulmonary delivery device of claim 6, wherein the control valve or control valves are independently connected to the control circuit, and wherein the control circuit is configured to open and close each valve a given number of times, per actuation.

8. The pulmonary delivery device of any preceding claim, wherein one or more of the vaporisers comprises a reservoir for retaining, in use, a quantity of the respective liquid and a conveyor adapted to convey, in use, the liquid from the reservoir to a heater, preferably wherein the conveyor comprises a wick extending between the interior of the reservoir and a heater or wherein the conveyor comprises a capillary tube extending between the reservoir and a heater.

9. The pulmonary delivery device of claim 8, wherein one or more of the vaporisers comprises an electric heater operatively connected to a battery.

10. The pulmonary delivery device of claim 9, further comprising a feedback circuit adapted to thermostatically regulate the temperature, or temperature profile of the heater or heaters.

11. The pulmonary delivery device of any preceding claim, further comprising an automatic switch adapted to switch the vaporiser on when a user inhales on the device, preferably wherein the switch comprises a pressure-activated switch associated with the outlet of the device.

12. The pulmonary delivery device of claim 11, wherein the controller is adapted to turn on the vaporiser in response to the triggering of only the first pressure-activated switch.

13. The pulmonary delivery device of any preceding claim, further comprising a wireless interface for operatively connecting the controller to an external computer, wherein the external computer is adapted to reprogram the controller, preferably wherein the external computer comprises a GUI permitting a user to select between different modes of operation of the device.

14. The pulmonary delivery device of claim 13, wherein one of the modes comprises a wean mode whereby the dose of a given medicament dispensed by the device can be reduced over time according to a configurable dose-time curve; and/or wherein one of the modes comprises a control program adapted to ensure that a desired quantity of medicament is administered over a period of time; and/or wherein the dose-per-actuation is dynamically controlled to facilitate, on average, a relatively even administration of the medicament over the time period; and/or wherein one of the modes comprises an upper limit adapted to prevent a maximum dose per unit time from being administered.

15. The pulmonary delivery device of any preceding claim, further comprising a display unit wherein the display unit comprises an LCD display adapted to provide a read-out of: the number of doses dispensed in total, the number of doses remaining, and/or an average number of doses dispensed per unit time.

## Patentansprüche

1. Vorrichtung zur pulmonalen Abgabe, die Folgendes umfasst: einen ersten Verdampfer, der zur Verdampfung einer Menge einer ersten Flüssigkeit geeignet ist, und einen zweiten Verdampfer, der zur Verdampfung einer Menge einer zweiten Flüssigkeit geeignet ist, wobei die zweite Flüssigkeit eine andere Flüssigkeit ist als die erste Flüssigkeit und wobei eine oder beide der ersten oder zweiten Flüssigkeit ein aktives Molekül, einen Hilfsstoff oder ein Medikament umfassen; und einen Auslass, über den bei Verwendung ein Anwender eine Mischung des ersten und zweiten Dampfs inhalieren kann, und durch Folgendes gekennzeichnet ist: einen programmierbaren Regler, der bei Verwendung zur automatischen Regelung des Verhältnisses der ersten und zweiten Flüssigkeit in der Mischung geeignet ist, um das Verhältnis der Flüssigkeiten auf einer dosisabhängigen Grundlage zu verändern.

2. Vorrichtung zur pulmonalen Abgabe nach Anspruch 1, wobei der Regler mit einer Anzahl von vom Anwender auswählbaren Betriebsmodi vorkonfiguriert ist und wobei die Vorrichtung einen Wahlschalter umfasst, der zum Wechseln des Reglers zwischen den verschiedenen Betriebsmodi geeignet ist, bevorzugt wobei vom Anwender auswählbare Betriebsmodi Folgendes umfassen: einen ersten Modus, in dem die Mischung 100 % der ersten Flüssigkeit und 0 % der zweiten Flüssigkeit umfasst, und einen zweiten Modus, in dem ein ausgewähltes Verhältnis der zwei Flüssigkeiten in der Mischung vorliegt.

3. Vorrichtung zur pulmonalen Abgabe nach Anspruch 1 oder Anspruch 2, wobei der erste Verdampfer immer aktiviert ist und wobei der zweite Verdampfer durch eine Taste der Vorrichtung wahlweise aktiviert wird.

4. Vorrichtung zur pulmonalen Abgabe nach Anspruch 1, 2 oder 3, wobei der Regler zur Ausgabe einer bestimmten Dosis von einer der Flüssigkeiten pro Inhalation geeignet ist und/oder wobei der Regler zur Ausgabe einer bestimmten kumulativen Dosis von einer der Flüssigkeiten pro Zeiteinheit geeignet ist und/oder wobei der Regler zur Ausgabe einer bestimmten Dosis von einer der Flüssigkeiten nur zu vorgegebenen Tageszeiten und zur Blockierung oder Verhinderung der Ausgabe der Flüssigkeit zu anderen Tageszeiten geeignet ist und/oder wobei der Regler zur Zulassung von unbeschränktem Verbrauch einer ersten der Flüssigkeiten, doch einem abgemessen und/oder dosierten Verbrauch einer zweiten der Flüssigkeiten geeignet ist.

5. Vorrichtung zur pulmonalen Abgabe nach Anspruch 1, wobei der Regler zur Einstellung des Verhältnisses der ersten und zweiten Flüssigkeit in der Mischung geeignet ist, um eine übermäßige oder unzureichende Verwendung der Vorrichtung auszugleichen.

6. Vorrichtung zur pulmonalen Abgabe nach einem vorstehenden Anspruch, wobei ein oder mehrere der Verdampfer einen Zerstäuber umfassen, bevorzugt wobei der Zerstäuber Folgendes umfasst: einen Vorratsbehälter, der eine Flüssigkeit unter Druck enthält, die durch eine Auslassöffnung gedrängt werden kann, um einen stabilen Dampf zu bilden, und ein Flussregelventil für die selektive Öffnung und Schließung der Auslassöffnung.

7. Vorrichtung zur pulmonalen Abgabe nach Anspruch 6, wobei das Regelventil oder die Regelventile unabhängig mit dem Regelkreis verbunden sind und wobei der Regelkreis zur Öffnung und Schließung von jedem Ventil für eine vorgegebene Anzahl von Malen pro Betätigung konfiguriert ist.

8. Vorrichtung zur pulmonalen Abgabe nach einem vorstehenden Anspruch, wobei ein oder mehrere der Verdampfer Folgendes umfassen: einen Vorratsbehälter für die Bewahrung einer Menge der jeweiligen Flüssigkeit bei Verwendung und ein Beförderungsmittel, das bei Verwendung zur Beförderung der Flüssigkeit von dem Vorratsbehälter zu einem Heizelement geeignet ist, bevorzugt wobei das Beförderungsmittel einen Docht umfasst, der sich zwischen dem Inneren des Vorratsbehälters und einem Heizelement erstreckt, oder wobei das Beförderungsmittel ein Kapillarrohr umfasst, das sich zwischen dem Vorratsbehälter und einem Heizelement erstreckt.

9. Vorrichtung zur pulmonalen Abgabe nach Anspruch 8, wobei ein oder mehrere der Verdampfer ein elektrisches Heizelement umfassen, das funktionsfähig mit einer Batterie verbunden ist.

10. Vorrichtung zur pulmonalen Abgabe nach Anspruch 9, die weiter einen Rückführkreis umfasst, der zur thermostatischen Regelung der Temperatur oder des Temperaturprofils des Heizelements oder der Heizelemente geeignet ist.

11. Vorrichtung zur pulmonalen Abgabe nach einem vorstehenden Anspruch, die weiter einen automatischen Schalter umfasst, der zum Anschalten des Verdampfers geeignet ist, wenn ein Anwender mit der Vorrichtung inhaliert, bevorzugt wobei der Schalter einen durch Druck aktivierten Schalter umfasst, der mit dem Auslass der Vorrichtung verbunden ist.

12. Vorrichtung zur pulmonalen Abgabe nach Anspruch 11, wobei der Regler zum Anschalten des Verdampfers als Reaktion auf die Auslösung des nur ersten durch Druck aktivierten Schalters geeignet ist.

13. Vorrichtung zur pulmonalen Abgabe nach einem vorstehenden Anspruch, die weiter eine drahtlose Schnittstelle für die funktionsfähige Verbindung des Reglers mit einem externen Rechner umfasst, wobei der externe Rechner zur Umprogrammierung des Reglers geeignet ist, bevorzugt wobei der externe Rechner eine GUI (grafische Benutzeroberfläche) umfasst, die einem Anwender ermöglicht, zwischen verschiedenen Betriebsmodi der Vorrichtung auszuwählen.

14. Vorrichtung zur pulmonalen Abgabe nach Anspruch 13, wobei einer der Modi einen Entwöhnungsmodus umfasst, wodurch die Dosis eines vorgegebenen Medikaments, das durch die Vorrichtung ausgegeben wird, über einen Zeitraum gemäß einer konfigurierbaren Dosis-Zeit-Kurve reduziert werden kann; und/oder wobei einer der Modi ein Kontrollprogramm umfasst, das zur Sicherstellung geeignet ist, dass eine gewünschte Menge an Medikament über eine Zeitdauer verabreicht wird; und/oder wobei die Dosierung pro Betätigung dynamisch geregelt wird, um im Durchschnitt eine relativ gleichmäßige Verabreichung des Medikaments über die Zeitdauer zu ermöglichen; und/oder wobei einer der Modi eine obere Grenze umfasst, die zur Verhinderung geeignet ist, dass eine maximale Dosis pro Zeiteinheit verabreicht wird.

15. Vorrichtung zur pulmonalen Abgabe nach einem vorstehenden Anspruch, die weiter eine Anzeigeeinheit umfasst, wobei die Anzeigeeinheit eine LCD-Anzeige umfasst, die zur Bereitstellung einer Auslesung von Folgenden geeignet ist: der Anzahl an Dosen, die insgesamt ausgegeben werden, der Anzahl an verbleibenden Dosen und/oder einer durchschnittlichen Anzahl an Dosen, die pro Zeiteinheit ausgegeben werden.

## Revendications

1. Appareil d'administration par voie pulmonaire comprenant : un premier vaporisateur adapté pour vaporiser une quantité d'un premier liquide, et un second vaporisateur adapté pour vaporiser une quantité d'un second liquide, le second liquide étant un liquide différent du premier liquide et dans lequel un ou les deux du premier ou du second liquide comprennent une molécule active, un excipient ou un médicament ; et une sortie par le biais de laquelle, en service, un utilisateur peut inhaler un mélange des première et seconde vapeurs et **caractérisé par** : un contrôleur programmable adapté pour commander automatiquement, en service, le rapport des premier et second liquides dans le mélange pour changer le rapport des liquides en fonction de la dose.

2. Dispositif d'administration par voie pulmonaire selon la revendication 1, dans lequel le contrôleur est préconfiguré avec un nombre de modes de fonctionnement sélectionnables par l'utilisateur, et dans lequel le dispositif comprend un commutateur de sélection adapté pour passer de manière cyclique le contrôleur entre les différents modes de fonctionnement, de préférence dans lequel des modes de fonctionnement sélectionnables par l'utilisateur comprennent un premier mode dans lequel le mélange comprend 100 % du premier liquide et 0 % du second liquide ; et un second mode dans lequel un rapport sélectionné des deux liquides est présent dans le mélange.

3. Dispositif d'administration par voie pulmonaire selon la revendication 1 ou la revendication 2, dans lequel le premier vaporisateur est toujours activé et le second vaporisateur est activé sélectivement par une touche du dispositif.

4. Dispositif d'administration par voie pulmonaire selon la revendication 1, 2 3, dans lequel le contrôleur est adapté pour distribuer une dose particulière d'un des liquides par inhalation ; et/ou dans lequel le contrôleur est adapté pour distribuer une dose cumulative particulière d'un des liquides par unité de temps ; et/ou dans lequel le contrôleur est adapté pour distribuer une dose particulière d'un des liquides uniquement à des moments prédéterminés de la journée, et inhiber ou empêcher la distribution dudit liquide à d'autres moments de la journée ; et/ou dans lequel le contrôleur est adapté pour autoriser une consommation illimitée d'un premier des liquides, mais une consommation mesurée et/ou dosée d'un second des liquides.

5. Dispositif d'administration par voie pulmonaire selon la revendication 1, dans lequel le contrôleur est adapté pour ajuster le rapport des premier et second liquides dans le mélange afin de compenser une surutilisation ou une sous-utilisation du dispositif.

6. Dispositif d'administration par voie pulmonaire selon n'importe quelle revendication précédente, dans lequel un ou plusieurs des vaporisateurs comprend un atomiseur, de préférence dans lequel l'atomiseur comprend un réservoir contenant un liquide sous pression qui peut être forcé à travers une ouverture de sortie pour former une vapeur stable et un clapet de commande d'écoulement pour ouvrir et fermer sélectivement l'ouverture de sortie.

7. Dispositif d'administration par voie pulmonaire selon la revendication 6, dans lequel le ou les clapets de commande sont connectés indépendamment au circuit de commande, et dans lequel le circuit de commande est configuré pour ouvrir et fermer chaque clapet un nombre de fois prédéterminé, par actionnement.

8. Dispositif d'administration par voie pulmonaire selon n'importe quelle revendication précédente, dans lequel un ou plusieurs des vaporisateurs comprend un réservoir pour renfermer, en service, une quantité du liquide respectif et un mécanisme d'acheminement adapté pour acheminer, en service, le liquide depuis le réservoir jusqu'à un élément chauffant, de préférence dans lequel le mécanisme d'acheminement comprend une mèche s'étendant entre l'intérieur du réservoir et un élément chauffant ou dans lequel le mécanisme d'acheminement comprend un tube capillaire s'étendant entre le réservoir et un élément chauffant.

9. Dispositif d'administration par voie pulmonaire selon la revendication 8, dans lequel un ou plusieurs des vaporisateurs comprend un élément chauffant électrique connecté opérationnellement à une batterie.

10. Dispositif d'administration par voie pulmonaire selon la revendication 9, comprenant en outre un circuit de rétroaction adapté pour réguler thermostatiquement la température, ou le profil de température du ou des éléments chauffants.

11. Dispositif d'administration par voie pulmonaire selon n'importe quelle revendication précédente, comprenant en outre un commutateur automatique adapté pour activer le vaporisateur quand un utilisateur aspire dans le dispositif, de préférence dans lequel le commutateur comprend un commutateur activé par pression associé à la sortie du dispositif.

12. Dispositif d'administration par voie pulmonaire selon la revendication 11, dans lequel le contrôleur est adapté pour activer le vaporisateur en réponse au déclenchement d'uniquement le premier commutateur activé par pression.

13. Dispositif d'administration par voie pulmonaire selon n'importe quelle revendication précédente, comprenant en outre une interface sans fil pour connecter opérationnellement le contrôleur à un ordinateur externe, l'ordinateur externe étant adapté pour reprogrammer le contrôleur, de préférence l'ordinateur externe comprenant une GUI permettant à un utilisateur de sélectionner différents modes de fonctionnement du dispositif.

14. Dispositif d'administration par voie pulmonaire selon la revendication 13, dans lequel un des modes comprend un mode de de sevrage dans lequel la dose d'un médicament donné distribuée par le dispositif peut être réduite dans le temps conformément à une courbe dose/temps configurable ; et/ou dans lequel un des modes comprend un programme de commande adapté pour garantir qu'une quantité souhaitée de médicament est administrée durant une période de temps ; et/ou dans lequel la dose par actionnement est commandée dynamiquement pour faciliter, en moyenne, une administration relativement régulière du médicament durant la période de temps ; et/ou dans lequel un des modes comprend une limite supérieure adaptée pour empêcher l'administration d'une dose maximale par unité de temps.

15. Dispositif d'administration par voie pulmonaire selon n'importe quelle revendication précédente, comprenant en outre une unité d'affichage, l'unité d'affichage comprenant un écran à cristaux liquides adapté pour permettre une lecture : du nombre total de doses distribuées, du nombre de doses restantes, et/ou d'un nombre moyen de doses distribuées par unité de temps.
